# EUROPEAN PATENT APPLICATION

(11) **EP 3 868 884 A1**
(43) Date of publication of application: **25.08.2021**
(21) Application number: 20158164.2
(22) Date of filing: 19.02.2020
(51) Int. Cl.: C12N 15/115

(54) **METHOD FOR SUBTYPING BLADDER CANCER USING APTAMERS**

(71) Applicant: Aarhus Universitet, 8000 Aarhus C (DK)
(72) Inventor: KJEMS, Jørgen, 8240 Risskov (DK); DUPONT, Daniel Miotto, 8520 Lystrup (DK)
(74) Representative: Plougmann Vingtoft a/s

(57) **Abstract**

The present invention relates to methods for distinguishing healthy subjects from subjects at risk of developing or having a non-invasive bladder cancer or an invasive bladder cancer using RNA aptamers. The invention also relates to composition and kits comprising the RNA aptamers and uses thereof.

SEQUENCE LISTING

<110>  Aarhus University

<120>  METHOD FOR SUBTYPING BLADDER CANCER USING APTAMERS

<130>  70984EP01

<160>  37

<170>  PatentIn version 3.5

<210>  1
<211>  36
<212>  RNA
<213>  Artificial Sequence

<220>
<223>  Aptamer

<400>  1
uuuccgcuga guaaagcgca gucgagucuu ccgacc                                36


<210>  2
<211>  36
<212>  RNA
<213>  Artificial Sequence

<220>
<223>  Aptamer

<400>  2
cuugucauca gaaacgcgcu aguuucgcau ucuguc                                36


<210>  3
<211>  36
<212>  RNA
<213>  Artificial Sequence

<220>
<223>  Aptamer

<400>  3
ucggcguugu cagaagaccg aaacuuagac uguguu                                36


<210>  4
<211>  36
<212>  RNA
<213>  Artificial Sequence

<220>
<223>  Aptamer

<400>  4
uuacgguacg gcuaaaacug cacgaccucu caggcc                                36


<210>  5
<211>  36
<212>  RNA

<213>  Artificial Sequence

<220>
<223>  Aptamer

<400>  5
uuuagaaguu gaucuaauuc uaacucagua cgucac                                36


<210>  6
<211>  36
<212>  RNA
<213>  Artificial Sequence

<220>
<223>  Aptamer

<400>  6
ugagauacca ggcggucaua ugacaccgaa cuaguc                                36


<210>  7
<211>  37
<212>  RNA
<213>  Artificial Sequence

<220>
<223>  Aptamer

<400>  7
uuuucccuuc ucaguaaggc agucguguuu caugacc                               37


<210>  8
<211>  36
<212>  RNA
<213>  Artificial Sequence

<220>
<223>  Aptamer

<400>  8
cgagcgguaa auccaagccg cacuucaguc cgucac                                36


<210>  9
<211>  36
<212>  RNA
<213>  Artificial Sequence

<220>
<223>  Aptamer

<400>  9
uaacgaauau gauccgauuc gcucucagca caucac                                36


<210>  10
<211>  36
<212>  RNA
<213>  Artificial Sequence

<220>

<223>  Aptamer

<400>  10
cuuaacauca gcacaaauuc uagugcgcau ucugcc                                36


<210>  11
<211>  36
<212>  RNA
<213>  Artificial Sequence

<220>
<223>  Aptamer

<400>  11
uugcgacgau uauugaaacc acacgucgcc ucaggu                                36


<210>  12
<211>  36
<212>  RNA
<213>  Artificial Sequence

<220>
<223>  Aptamer

<400>  12
uuuuagcucu gaucuaagcu aaacucacgc cauccc                                36


<210>  13
<211>  36
<212>  RNA
<213>  Artificial Sequence

<220>
<223>  Aptamer

<400>  13
gcaucaccgc ucgacuuaca ucgacccgug uaaaac                                36


<210>  14
<211>  36
<212>  RNA
<213>  Artificial Sequence

<220>
<223>  Aptamer

<400>  14
uucgcgcaga gcuucagguu ucaauuuaac ccuuuc                                36


<210>  15
<211>  36
<212>  RNA
<213>  Artificial Sequence

<220>
<223>  Aptamer

<400>  15

aaauuucccu aaauuguagg cagguacacc guaccc 36

<210> 16
<211> 36
<212> RNA
<213> Artificial Sequence

<220>
<223> Aptamer

<400> 16
gucuaacugu cggagguacu gucaacacau acgaug 36

<210> 17
<211> 36
<212> RNA
<213> Artificial Sequence

<220>
<223> Aptamer

<400> 17
acgcgucuug gugacgcuuu ugugaaucua agccac 36

<210> 18
<211> 36
<212> RNA
<213> Artificial Sequence

<220>
<223> Aptamer

<400> 18
uuaccuacuc cguacauucg gauggugccg aacguu 36

<210> 19
<211> 36
<212> RNA
<213> Artificial Sequence

<220>
<223> Aptamer

<400> 19
aguguaggaa cuuucggacc uccaucauaa gcaugc 36

<210> 20
<211> 36
<212> RNA
<213> Artificial Sequence

<220>
<223> Aptamer

<400> 20
gcuuaucgug ucuugguacu gucagcacau acuaug 36

<210>  21
<211>  36
<212>  RNA
<213>  Artificial Sequence

<220>
<223>  Aptamer

<400>  21
gguuucaaug ucccgguacu gucaucacau acaaug                                36


<210>  22
<211>  36
<212>  RNA
<213>  Artificial Sequence

<220>
<223>  Aptamer

<400>  22
agcaacuacc augacaacgu guuagucggu cgaaau                                36


<210>  23
<211>  36
<212>  RNA
<213>  Artificial Sequence

<220>
<223>  Aptamer

<400>  23
aacguuaaau gagccaagcg acuugcauac uuggac                                36


<210>  24
<211>  36
<212>  RNA
<213>  Artificial Sequence

<220>
<223>  Aptamer

<400>  24
caaagccgac augcuggugu ucaugaauac accauc                                36


<210>  25
<211>  36
<212>  RNA
<213>  Artificial Sequence

<220>
<223>  Aptamer

<400>  25
uuaggugcuc ugaacuuuac aucggaacua acguug                                36


<210>  26
<211>  37
<212>  RNA

<213>  Artificial Sequence

<220>
<223>  Aptamer

<400>  26
aguacaccgu aacacuacgg cuuaucauaa gcaugac                                37


<210>  27
<211>  35
<212>  RNA
<213>  Artificial Sequence

<220>
<223>  Aptamer

<400>  27
uaaagcgucg ggccguacug ucaauacaua caaug                                  35


<210>  28
<211>  36
<212>  RNA
<213>  Artificial Sequence

<220>
<223>  Aptamer

<400>  28
guucgaucgg cguuauaaua ccacgcgacc cuugac                                 36


<210>  29
<211>  36
<212>  RNA
<213>  Artificial Sequence

<220>
<223>  Aptamer

<400>  29
gcggugcuuc gcugcgacau ccgcgaaacc aacguc                                 36


<210>  30
<211>  36
<212>  RNA
<213>  Artificial Sequence

<220>
<223>  Aptamer

<400>  30
   agugugaacg aacaacguuc gucccaucau aagcau                                  36


<210>  31
<211>  36
<212>  RNA
<213>  Artificial Sequence

<220>

<223>  Aptamer

<400>  31
ugaguggugc uccuacaacg caggaaccuc aaugcu                                36


<210>  32
<211>  36
<212>  RNA
<213>  Artificial Sequence

<220>
<223>  Aptamer

<400>  32
ccgaccuuag ucaaucacuu cguucgauga auagca                                36


<210>  33
<211>  36
<212>  RNA
<213>  Artificial Sequence

<220>
<223>  Aptamer

<400>  33
ucguucgauc ucucgaguuc ucagagcgac cuuuau                                36


<210>  34
<211>  36
<212>  RNA
<213>  Artificial Sequence

<220>
<223>  Aptamer

<400>  34
agcacaaaau uacgcaaccu ugggacuuag cugauu                                36


<210>  35
<211>  44
<212>  DNA
<213>  Artificial Sequence

<220>
<223>  Aptamer

<400>  35
cgcggatcct aatacgactc actatagggg ccaccaacga catt                       44


<210>  36
<211>  79
<212>  DNA
<213>  Artificial Sequence

<220>
<223>  Aptamer

<220>
<221>  misc_feature
<222>  (26)..(62)
<223>  n is a, c, g, or t

<400>  36
gatccatggg cactatttat atcaacnnnn nnnnnnnnnn nnnnnnnnnn nnnnnnnnnn      60

nnaatgtcgt tggtggccc                                                   79


<210>  37
<211>  38
<212>  DNA
<213>  Artificial Sequence

<220>
<223>  Aptamer

<400>  37
cccgacaccc gcggatccat gggcactatt tatatcaa                              38

## Description

### Technical field of the invention

The present invention relates to RNA aptamers able to identify subjects considered at risk of having bladder cancer. In particular, the present invention relates to RNA aptamers able to distinguish subjects having non-invasive bladder cancer from subjects having invasive bladder cancer and to stage bladder cancer patients.

### Background of the invention

The millions of molecules present in blood reflect the ongoing and historic activities for each human being and provides information about our gender, age, health condition, diseases etc. There is currently a high demand for novel analysis tools capable of monitoring the health status of individuals.
In Denmark, around 2000 people are diagnosed with bladder cancer every year and 20,000 currently live with the disease. With an estimated 430,000 new cases and 165,000 related deaths worldwide every year, bladder cancer is the ninth most common cancer type worldwide in general, and due to a strong male predominance, the fifth most common among men. Most cases of newly diagnosed bladder cancers are of benign type (∼75%, non-invasive stage Ta and T1) and the prognosis good for this group with current treatment (TURBT transurethral resection and intravesical chemotherapy). However, relapse is frequent (>35% every year) and lifelong monitoring therefore necessary. Standard diagnosis and surveillance includes periodic cystoscopy (visual assessment of the bladder by inserting a thin tube with a camera through the urinary tract) and urine cytology (detection of abnormal cells). Although considered as a mild procedure, complications of cystoscopy can include urinary tract infection, anastesia risks, urination problems and pain, as well as reduction in sexual function. In addition, particularly the life-long follow-up surveillance is a considerable financial burden for the healthcare system as every periodic examination requires a doctor and anastesia personel.

Being able to reduce the number of cystoscopic analyses and replace them with less invasive and less costly alternatives, would be of considerable value freeing up resources in the health care sector. In Denmark 15,000-20,000 cystoscopies are performed every year and the total for EU and the U.S. is more than 1 mio. (cost per cystoscopy is 1,500-2,500 DKK).
The 5-year survival rate for bladder cancer patients depends dramatically on the stage of the cancer at the time of diagnosis. It is 95% for non-muscle invasive (Ta) cancer, declining to 69% for muscle invasive (T2-4) and further to 5% when the cancer has metastasized. Hence, there is also a need for new methods that allow the possibility for early detection and screening, particular for high-risk groups such as smokers. The greatest risk factor for bladder cancer is tobacco smoke, accounting for about 50% of all cases.

Finally, there is an unmet need for novel tests, which are able to predict the prognosis of bladder cancer patients and their response to therapy, in order to tailor medication individually and help focus expensive treatment to those patients that will benefit, while sparing those who won't for unnecessary side effects. Currently, among the cancer patients with benign tumours it is not possible to predict, which patients that will experience relapse, which tumours that are prone to become malignant, and which patients who will benefit from general preventive treatment.

Today, plasma proteins are the most assayed class of laboratory analytes in the clinic providing important information about disease and health condition. Laboratory testing encompasses the monitoring of enzymatic activities and antibody-based immunoassays. More than 100 plasma or serum tests have been FDA-cleared or FDA-approved, mainly for the abundant, functional proteins, followed by tissue leakage markers, immunoglobulins and tumour markers. However, most of the assays are decades old, and the current introduction rate of new markers is less than two each year. Furthermore, the tests usually only address single targets. LC-MS/MS can perform multiplexed analysis, but although it has become much more robust, it is still far from perfect for routine biomarker identification and clinical application.

Aptamers are short nucleic acid strands (RNA/DNA), which can form a variety of sequence-dependent shapes, capable of specific molecular recognition of almost any target. Screening of huge random-sequence nucleic acid libraries allows the identification of aptamer shapes for virtually any type of target, including proteins. In terms of binding characteristics (specificity and affinity), aptamers resemble antibodies. To increase functionality, aptamers may be constructed using modified unnatural nucleotides.

Hence, an improved method to subtype bladder cancers would be advantageous, and in particular a more efficient and/or reliable non-invasive method to subtype bladder cancers would be advantageous.

### Summary of the invention

The present invention relates to a multi-component profiling technology capable of extracting information from the molecular signature of highly complex samples, such as blood, using (RNA) aptamers and machine learning. As a proof of concept, bladder cancer blood samples were analyzed and signatures of healthy versus sick patients as well as cancer stage, i.e. non-muscle invasive (Ta) versus muscle invasive (T2-T4) were determined (see examples 1 and 2).

In addition, 34 single aptamers from the sensor panels were able to detect bladder cancer and bladder cancer stage individually. Hence, aptamer profiling and individual aptamers derived from the analyses may be used as a point-of-care test package for bladder cancer screening, diagnosis and prognosis determination, relapse monitoring and therapy response prediction (see examples 1 and 2).

Thus, an object of the present invention relates to the provision of a method to subtype and/or diagnose bladder cancers.

In particular, it is an object of the present invention to provide single aptamers and aptamer subsets, which can be used in such methods.

Thus, one aspect of the invention relates to a method for determining the risk of a subject for having or developing bladder cancer or for staging a bladder cancer, the method comprising
a) determining for a biological sample from a subject, an RNA aptamer binding profile,
b) comparing the RNA aptamer binding profile to one or more RNA aptamer binding reference profiles;
wherein the comparison to the one or more RNA aptamer binding references profiles, is indicative of the subject being at risk of having or developing invasive bladder cancer or having or developing non-invasive bladder cancer;
or
wherein the comparison to the one or more RNA aptamer binding references profiles, is indicative of a stage of a bladder cancer;
**characterized in** that in step a) the biological sample is mixed with at least one RNA aptamer comprising a sequence selected from the group consisting of SEQ ID NO: 4 and SEQ ID NO's: 1-3 and 5-34.

Another aspect of the present invention relates to a method for determining an increased, unchanged or decreased risk of developing or having bladder cancer or determining a change in a bladder cancer stage for a subject, the method comprising
- determining in a first biological sample from the subject, a first RNA aptamer binding profile;
- determining in a second corresponding biological sample from the subject, obtained at a later time point than said first sample, an second RNA aptamer binding profile;
wherein, a determined second RNA aptamer binding profile in the second sample different from the first RNA aptamer binding profile in the first sample, is indicative of an increased or decreased risk of developing or having bladder cancer or indicative of the bladder cancer stage has changed for the subject;
or
wherein, a determined second RNA aptamer binding profile in the second sample equal to the determined first RNA aptamer binding profile in the first sample is indicative of an unchanged risk of risk of developing or having bladder cancer or that the bladder cancer stage has not changed for the subject;
**characterized in** that the aptamer binding profiles include at least one RNA aptamer comprising a sequence selected from the group consisting of SEQ ID NO: 4 and SEQ ID NO's: 1-3 and 5-34.

A further aspect relates to a method for determining the effect of a treatment protocol against bladder cancer for a subject, the method comprising
- determining in a first biological sample (1) from the subject, a first RNA aptamer binding profile;
- determining in a second corresponding biological sample from the subject, obtained at a later time point than said first sample, an second RNA aptamer binding profile;
wherein the treatment protocol has been initiated or completed before the sampling of the first sample or initiated, continued or completed between the sampling of the first sample and the second sample; and
wherein, determined overall higher frequencies of the RNA aptamers in the second sample compared the determined frequencies of the RNA aptamers in the first sample, is indicative of the treatment protocol being efficient against the bladder cancer;
or
wherein, determined overall lower or equal frequencies of the RNA aptamers in the second sample compared the determined frequencies of the RNA aptamers in the first sample, is indicative of the treatment protocol being inefficient against the bladder cancer;
**characterized in** that the aptamer binding profiles include at least one RNA aptamer comprising a sequence selected from the group consisting of SEQ ID NO: 4 and SEQ ID NO's: 1-3 and 5-34.

Yet another aspect of the present invention is to provide a kit of parts comprising
- at least one RNA aptamer selected from the group consisting SEQ ID NO: 4 and SEQ ID NO's: 1-3 and 5-34;
- one or more primers for amplifying the aptamers;
- one or more enzymes for amplifying the aptamers;
- a solid support for sample capture, such as beads; and
- optionally, instructions for using the kit in a method according to the invention.

A further aspect relates to a composition comprising an RNA aptamer comprising an (aptamer) sequence selected from the group consisting of SEQ ID NO: 4 and SEQ ID NO's: 1-3 and 5-34.

Still another aspect of the present invention relates to the use of a kit according to the invention or a composition according to the invention in a method for determining the risk of a subject for having or developing non-invasive bladder cancer or invasive bladder cancer or for staging a bladder cancer.

### Brief description of the figures

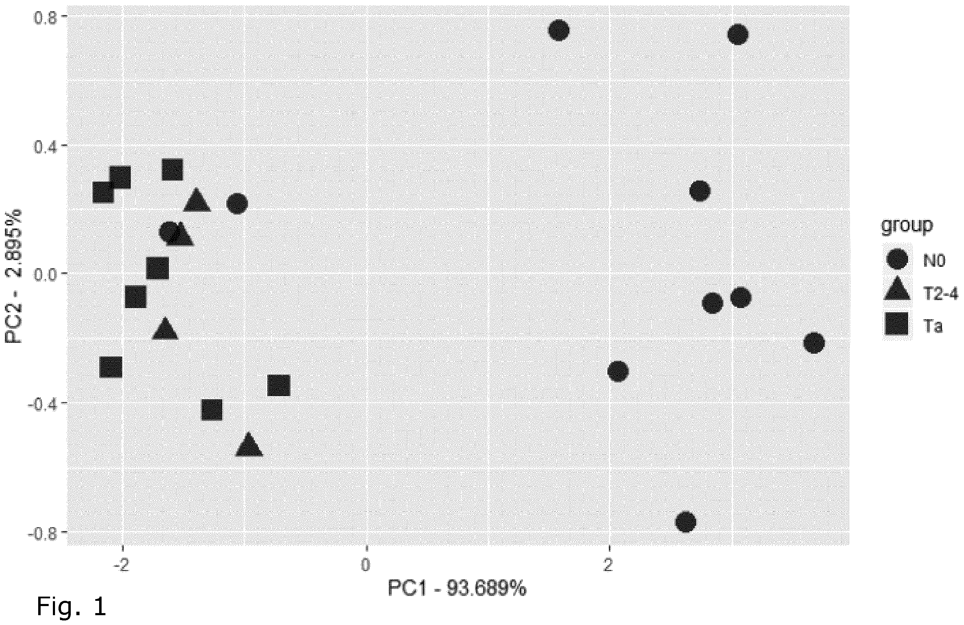

Figure 1 shows the characterisation of 10 samples from healthy individuals (N0, circles), 8 samples from bladder cancer patients with stage Ta (squares), and 4 bladder cancer patient samples with stage T2-4 (triangles) using a PCA analysis of the levels of aptamers (SEQ ID NO's: 1-15) after one round of branched selection of the samples.

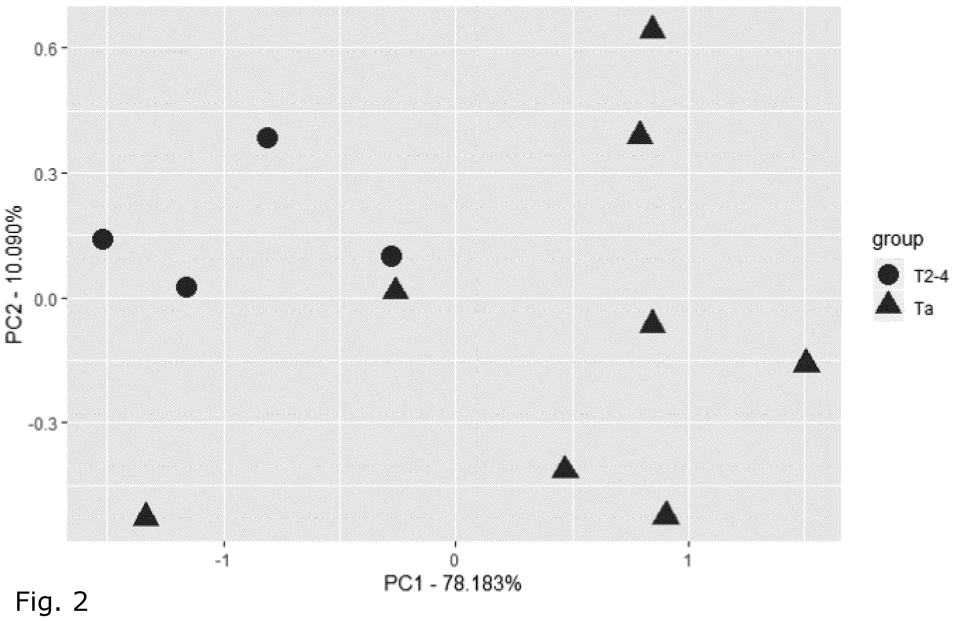

Figure 2 shows characterization of 8 samples from bladder cancer patients with stage Ta (squares), and 4 bladder cancer patient samples with stage T2-4 (triangles) using a PCA analysis of the levels of aptamers SEQ ID NO's: 16-33 after one round of branched selection of the samples.

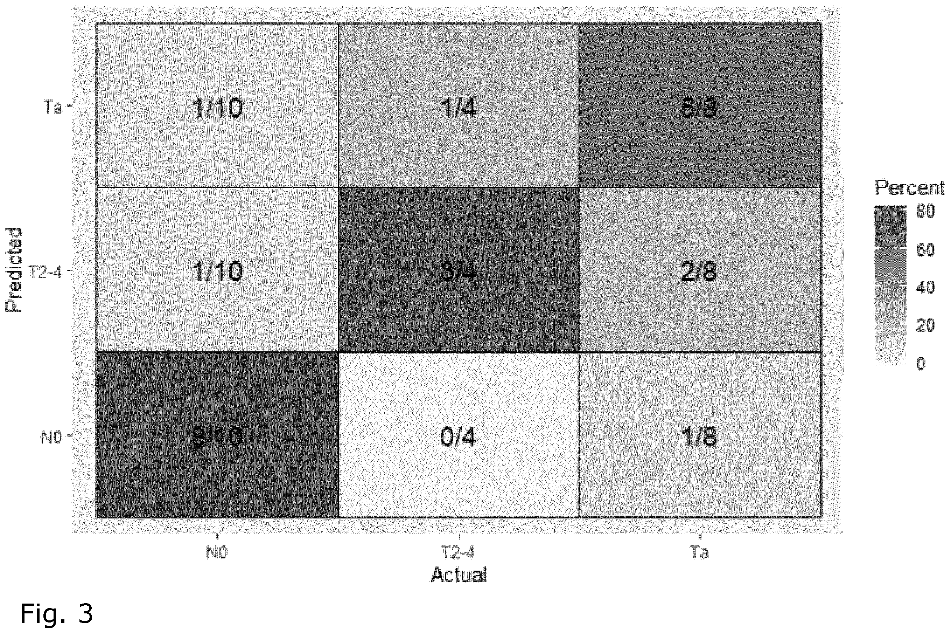

Figure 3 shows confusion matrix result for the partial least squares analysis of 10 samples from healthy individuals (N0), 8 samples from non-muscle invasive bladder cancer patients (Ta), and 4 samples from muscle-invasive bladder cancer patient (T2-4) using 33 aptamers (SEQ ID NO: 1-33) after one round of branched selection of the samples.

The present invention will now be described in more detail in the following.

### Detailed description of the invention

### Definitions

Prior to discussing the present invention in further details, the following terms and conventions will first be defined:

### Aptamer

In the present context, the term "aptamer", refers to oligonucleotides that resembles antibodies in their ability to act as ligands and bind to analytes. Aptamers may in general e.g. comprise natural DNA nucleotides, natural RNA nucleotides, modified DNA nucleotides, modified RNA nucleotides, or a combination thereof. In the present invention RNA aptamers are used which may comprise one or more modifications. Examples of modifications are artificial nucleotides or artificial bonds selected from the group consisting of 2'-F-pyrimidine, 2'-NH2-pyrimidine, 2'-O-Me, LNA (locked nucleic acid), 4'-S, TNA (threose nucleic acid), FANA (fluoroarabinonucleotide) and HNA (1,5-anhydrohexitol nucleic acid).

An advantage of aptamers compared to antibodies is that they may easily be sequenced and amplified after isolation.

### Relative frequency

The term "relative frequency" as used herein, with respect to an aptamer, refers to the copy number of that aptamer in a sample of sequences divided by the total number of sequences observed.

The term "change in relative frequency" as used herein, with respect to an aptamer, refers to the relative frequency of that aptamer in a sample compared to the relative frequency of the same aptamer in e.g. a reference sample.

### RNA aptamer binding profile

In the present context, the term "RNA aptamer binding profile" refers to the aptamers, number/frequency of the aptamers and/or relative frequency of the one or more aptamers present in a sample. For the method of the invention an "RNA aptamer binding profile" is preferably a profile of the aptamers, which bind to the components of a biological sample, said components being captured on a surface, such as a bead.

### Bladder cancer

Bladder cancer is any of several types of cancer arising from the tissues of the urinary bladder. It is a disease in which cells grow abnormally and have the potential to spread to other parts of the body.

Currently, the best diagnosis of the state of the bladder is by way of cystoscopy, which is a procedure in which a flexible or rigid tube (called a cystoscope) bearing a camera and various instruments is introduced into the bladder through the urethra.

In the present context "bladder cancer" are scored according to the TNM staging system approved 05/2019 (https://www.cancer.net/cancer-types/bladder-cancer/stages-and-grades).

"Non-invasive bladder cancer" refers to stages Ta-T1 according to the TNM staging system approved 05/2019.
Ta: This refers to non-invasive papillary carcinoma. This type of growth often is found on a small section of tissue that easily can be removed with TURBT.
Tis: This stage is carcinoma in situ (CIS) or a "flat tumour". This means that the cancer is only found on or near the surface of the bladder. The doctor may also call it non-muscle-invasive bladder cancer, superficial bladder cancer, or non-invasive flat carcinoma. This type of bladder cancer often comes back after treatment, usually as another non-invasive cancer in the bladder.
T1: The tumour has spread to the connective tissue (called the lamina propria) that separates the lining of the bladder from the muscles beneath, but it does not involve the bladder wall muscle.

"Invasive bladder cancer" refers to stages T2-T4 according to the TNM staging system approved 05/2019.
T2: The tumour has spread to the muscle of the bladder wall.
   - T2a: The tumour has spread to the inner half of the muscle of the bladder wall, which may be called the superficial muscle.
   - T2b: The tumour has spread to the deep muscle of the bladder (the outer half of the muscle).
T3: The tumour has grown into the perivesical tissue (the fatty tissue that surrounds the bladder).
   - T3a: The tumour has grown into the perivesical tissue, as seen through a microscope.
   - T3b: The tumour has grown into the perivesical tissue macroscopically. This means that the tumour(s) is large enough to be seen during imaging tests or to be seen or felt by the doctor.
T4: The tumour has spread to any of the following: the abdominal wall, the pelvic wall, a man's prostate or seminal vesicle (the tubes that carry semen), or a woman's uterus or vagina.
   - T4a: The tumour has spread to the prostate, seminal vesicles, uterus, or vagina.
   - T4b: The tumour has spread to the pelvic wall or the abdominal wall.

"Healthy subjects" refers to stage T0 according to the TNM staging system approved 05/2019.
T0 (T plus zero): There is no evidence of a primary tumor in the bladder.

Specific staging and grading of bladder cancer may vary from country to country and many different protocols exists.

### Subject

The term "subject" refers to a warm-blooded animal, preferably a human, a pet or livestock. As used herein, the terms "pet" and "livestock" include, but are not limited to, dogs, cats, guinea pigs, rabbits, pigs, cattle, sheep, goats, horses and poultry. In some embodiments, the subject is a male or female subject. In some embodiments, the subject is an adult (a subject of the age of 18 or above). In another embodiment, the subject is a child (a subject below the age of 18).

In an embodiment, the subject is a subject is considered at risk of having or developing non-invasive bladder cancer or invasive bladder cancer.

### Reference level / reference profile

In the context of the present invention, the term "reference level" or "reference profile" relates to a standard in relation to a quantity, which other values or characteristics can be compared to.

In one embodiment of the present invention, it is possible to determine a reference level/profile by investigating the binding profile for the aptamers according to the invention in (blood) samples from reference subjects. Such as healthy subject, subject diagnosed with non-invasive bladder cancer or diagnosed with invasive bladder cancer. By applying different statistical means, such as multivariate analysis, one or more reference levels can be calculated.

Based on these results a cut-off may be obtained that shows the relationship between the level(s) detected and patients at risk. The cut-off can thereby be used to determine the whether a subject should be considered at risk of having or developing non-invasive bladder cancer or invasive bladder cancer.

### Risk Assessment

The present inventors have successfully developed a new method to predict the risk for a subject for having or developing non-invasive bladder cancer or invasive bladder cancer. The results presented in the examples show that the described RNA aptamers appear to be able to function as tools to determine whether a subject is at risk of having or developing non-invasive bladder cancer or invasive bladder cancer.

To determine whether a patient has an increased risk of having or developing non-invasive bladder cancer or invasive bladder cancer, a cut-off must be established. This cut-off may be established by the laboratory, the physician or on a case-by-case basis for each patient.

The cut-off level could be established using a number of methods, including: multivariate statistical tests (such as partial least squares discriminant analysis (PLS-DA), random forest, support vector machine, Ordinary least square (OLS) etc.), percentiles, mean plus or minus standard deviation(s); median value; fold changes and machine learning.

The multivariate discriminant analysis and other risk assessments can be performed on the free or commercially available computer statistical packages (SAS, SPSS, Matlab, R, Python etc.) or other statistical software packages or screening software known to those skilled in the art.

As obvious to one skilled in the art, in any of the embodiments discussed above, changing the risk cut-off level could change the results of the discriminant analysis for each patient.

Statistics enable evaluation of the significance of each determined aptamer level/frequency. Commonly used statistical tests applied to a data set include t-test, f-test or even more advanced tests and methods of comparing data. Using such a test or method enables the determination of whether two or more samples are significantly different or not.

The significance may be determined by the standard statistical methodology known by the person skilled in the art.

The chosen reference level may be changed depending on the mammal/subject for which the test is applied.

Preferably, the subject according to the invention is a human subject, such as a male or female human subjects, preferably a male.

The chosen reference level may be changed if desired to give a different specificity or sensitivity as known in the art. Sensitivity and specificity are widely used statistics to describe and quantify how good and reliable a biomarker or a diagnostic test is. Sensitivity evaluates how good a biomarker or a diagnostic test is at detecting a disease, while specificity estimates how likely an individual (i.e. control, patient without disease) can be correctly identified as not sick.
Several terms are used along with the description of sensitivity and specificity; true positives (TP), true negatives (TN), false negatives (FN) and false positives (FP). If a disease is proven to be present in a sick patient, the result of the diagnostic test is considered to be TP. If a disease is not present in an individual (i.e. control, patient without disease), and the diagnostic test confirms the absence of disease, the test result is TN. If the diagnostic test indicates the presence of disease in an individual with no such disease, the test result is FP. Finally, if the diagnostic test indicates no presence of disease in a patient with disease, the test result is FN.

### Sensitivity

Sensitivity = TP/ (TP + FN) = number of true positive assessments/ number of all samples from patients with disease.
As used herein the sensitivity refers to the measures of the proportion of actual positives which are correctly identified as such - in analogy with a diagnostic test, i.e. the percentage of people having non-invasive bladder cancer or invasive bladder cancer who are identified as having non-invasive bladder cancer or invasive bladder cancer.

### Specificity

Specificity= TN/ (TN+ FP) = number of true negative assessments/ number of all samples from controls.
As used herein the specificity refers to measures of the proportion of negatives which are correctly identified - i.e. the percentage of mammals or people being healthy who are identified as being healthy. The relationship between both sensitivity and specificity can be assessed by the ROC curve. This graphical representation helps to decide the optimal model through determining the best threshold -or cut-off for a diagnostic test or a biomarker candidate.

As will be generally understood by those skilled in the art, methods for screening are processes of decision-making and therefore the chosen specificity and sensitivity depend on what is considered to be the optimal outcome by a given institution/clinical personnel.

It would be obvious for a person skilled in the art that it may be advantageous to select a higher sensitivity at the expense of lower specificity in most cases, to identify as many patients with disease as possible.
In a preferred embodiment, the invention relates to a method with a high specificity, such as at least 70%, such as at least 80%, such as at least 90%, such as at least 95%, such as 100%.

In another preferred embodiment, the invention relates to a method with a high sensitivity, such as at least 80%, such as at least 90%, such as 100%.

### Method for determining the risk of a subject for having or developing bladder cancer or for staging a bladder cancer

As outlined above, the present invention relates to the identification of a set of RNA aptamers, which may be used in relation to bladder cancer diagnostics. Thus, a first aspect of the invention relates to a method for determining the risk of a subject for having or developing bladder cancer or for staging a bladder cancer, the method comprising
a) determining for a biological sample from a subject, an RNA aptamer binding profile,
b) comparing the RNA aptamer binding profile to one or more RNA aptamer binding reference profiles;
wherein the comparison to the one or more RNA aptamer binding references profiles, is indicative of the subject being at risk of having or developing invasive bladder cancer or having or developing non-invasive bladder cancer;
or
wherein the comparison to the one or more RNA aptamer binding references profiles, is indicative of a stage of a bladder cancer;
**characterized in** that in step a) the biological sample is mixed with at least one RNA aptamer comprising a sequence selected from the group consisting of SEQ ID NO: 4 and SEQ ID NO's: 1-3 and 5-34.

The different aptamers can be used both to distinguish healthy subjects from subjects having bladder cancer and to stage e.g. invasive bladder cancers (see example 2).

The method may find use, even when the source of the biological sample varies. Thus, in an embodiment, the biological sample is selected from the group consisting of a blood sample, such as blood serum or blood plasma, a urine sample, a CNF sample, saliva, and a tissue sample. In the example section, blood samples have been tested (plasma), but other sample types will likely also work, such as urine samples or cancer tissue samples. In a preferred embodiment, the biological sample is a blood plasma sample.

How the RNA aptamer binding profile will differ from a reference profile, of course depends on the reference. In an embodiment, an RNA aptamer binding profile different from one or more reference RNA aptamer profiles, is indicative of the subject being at risk of having or developing invasive bladder cancer or having or developing non-invasive bladder cancer or being indicative of a stage of a bladder cancer. This will likely be the case if a reference profile(s) is from a healthy subject(s).

In an opposite embodiment, an RNA aptamer binding profile identical or similar to one or more reference RNA aptamer profiles, is indicative of the subject being at risk of having or developing invasive bladder cancer or having or developing non-invasive bladder cancer or being indicative of a stage of a bladder cancer. This will likely be the case if a reference profile is from a subject(s) suffering from bladder cancer, such as non-invasive bladder cancer or invasive bladder cancer.

To be able to further improve the assay, and to quantify (determine frequency) the level of each aptamer, non-descriptive RNA aptamers may be included in assay. Thus, in an embodiment, in step a), a pool of non-descriptive RNA aptamers are included. In this way, it is possible to determine relative levels/changes for individual aptamers, compared to the non-descriptive RNA aptamers (see example 2).

For diagnostic assays, it is an advantage if only small amount of sample material is required. For blood samples it would be an advantage if the sample could be obtained e.g. from the fingertip. Thus, in an embodiment, the volume of the biological sample is in the range 0.1 µl to 50 µl, such as 0.1 µl to 25 µl, such as 0.1 µl to 10 µl, such as 0.1 µl to 5 µl, such as 0.1 µl to 3 µl, such as 0.5 µl to 2, or such as around 1 µl, preferably a blood plasma sample.

It may also be an advantage if non-relevant abundant proteins could be removed from the biological sample before mixing with the aptamers. Thus, in another embodiment, the biological sample is protein depleted, such as by being passed through a protein depletion column, such as a high select Top14 abundant protein depletion column before mixing with the RNA aptamer(s).

Step a) in the method of the invention could also include additional steps. Thus, in an embodiment, step a) comprises:
- a1) incubating the biological sample with a surface, thereby enabling coupling of molecules (such as through amin-coupling) in the biological sample to the surface;
- a2) optionally, washing the surface, thereby removing unbound molecules;
- a3) incubating the surface comprising bound molecules, with at least one RNA aptamer comprising a sequence selected from the group consisting of SEQ ID NO's: 1-34;
- a4) optionally, washing the surface, thereby removing unbound aptamers;
- a5) optionally, releasing the aptamers bound to the surface; and
- a6) determining the identity and optionally frequency of bound aptamers, thereby obtaining an RNA aptamer binding profile.

As also outlined above, a step A0 could be protein depletion of the sample, such as by passing the biological sample through a protein depletion column.

In yet an embodiment, in step a1), the surface is selected from the group consisting of beads, such as surface-reacted beads, columns, such as surface-reacted columns, preferably surface-reacted beads.

In a further embodiment, step a5), the identity, and optionally (relative) frequency, of bound aptamers to the surface is determined by one or more methods selected from the group consisting of sequencing, such as next-generation sequencing, third generation sequencing, such as Nanopore technology, such as Oxford Nanopore.

In yet a further embodiment, the frequency of the identified aptamers is also determined, such as the relative frequency of the identified aptamers is determined compared to a reference level. The reference level of the frequency may be relative to non-descriptive RNA aptamers included in the assay.

The method of the invention preferably includes more than one RNA aptamer, albeit individual aptamers are also useful. Thus, in an embodiment, the biological sample is mixed with at least five different RNA aptamers comprising a sequence selected from the group consisting of SEQ ID NO's: 1-34, such as at least 10 different RNA aptamers, such as at least 20 different RNA aptamers, such as at least 30 different RNA aptamers, or such as 34 different RNA aptamers.

In a preferred embodiment, the method comprises at least SEQ ID NO: 4. SEQ ID NO: 4 seems to be the aptamer providing the highest predictive value.

In an embodiment, the method comprises at least SEQ ID NO's: 4 and 34.

In an embodiment, the method comprises at least one of SEQ ID NO's: 1-15, 34, with the proviso that the method is for determining the risk for a subject for having bladder cancer, such as at least 5 of SEQ ID NO's: 1-15, such as at least 10 of SEQ ID NO's: 1-15, or such as all of SEQ ID NO's: 1-15, preferably comprising SEQ ID NO: 4 (Figure 1), more preferably comprising at least SEQ ID NO's: 4 and 34.

In an embodiment, the staging of bladder cancer is staging a non-invasive bladder cancer, such as staging as a Ta or T1 bladder cancer or staging an invasive bladder cancer, such as staging as a T2, T3 or T4 bladder cancer. As shown in example 2, the method of the invention is able stage such bladder cancers.

In another embodiment, the method comprises at least one of SEQ ID NO's: 16-33, with the proviso that the method is for subtyping bladder cancer, such as non-invasive vs invasive bladder cancer, or such as Ta-T1 vs T2-T4. As shown in example 2 (Figure 2), these aptamers works well for this subtyping/staging bladder cancers. In an embodiment, the method comprises at least 5 of SEQ ID NO's: 16-33, such as at least 10 of SEQ ID NO's: 16-33 or such as all of SEQ ID NO's: 16-33.

In another embodiment, the method comprises at least one of SEQ ID NO's: 16-33, such as at least 10 of SEQ ID NO's: 16-33 or such as all of SEQ ID NO's: 16-33, with the proviso that the method is for subtyping invasive bladder cancer, such as at least T2 vs T3 vs T4, or other categories according to the TNM staging system (Figure 2).

The RNA aptamer binding reference profile can be obtained in different ways. In an embodiment, the RNA aptamer binding reference profile is obtained from reference material, such as selected from the group consisting of corresponding biological samples obtained from healthy subjects, subjects diagnosed with non-invasive bladder cancers, subjects diagnosed with invasive bladder cancer, bladder cancer cell lines or bladder cancer tumour samples.

For the individual aptamers lower or higher frequencies are related to bladder cancers compared to healthy subjects.

Thus, in an embodiment,
- a lower relative frequency of one or more of SEQ ID NO's: 25, 29 and/or 31 is indicative of invasive bladder cancer (see figure 2); and/or
- a higher relative frequency of one or more of SEQ ID NO's: 16-24, 26-28, 30 and/or 32-33 is indicative of invasive bladder cancer (see figure 2); and/or
- a lower relative frequency of one or more of SEQ ID NO's: 1-15 is indicative of bladder cancer (see figure 1); and/or
- a higher relative frequency of SEQ ID NO: 34 is indicative of bladder cancer.

### Monitoring development of bladder cancer

The method of the invention may also be used for following the development of bladder cancer for a subject. Such a monitoring whether a subject develops bladder cancer or monitoring whether a non-invasive bladder cancer develops into an invasive bladder cancer. Thus, an aspect of the invention relates to a method for determining an increased, unchanged or decreased risk of developing or having bladder cancer or determining a change in a bladder cancer stage for a subject, the method comprising
- determining in a first biological sample from the subject, a first RNA aptamer binding profile;
- determining in a second corresponding biological sample from the subject, obtained at a later time point than said first sample, an second RNA aptamer binding profile;
wherein, a determined second RNA aptamer binding profile in the second sample different from the first RNA aptamer binding profile in the first sample, is indicative of an increased or decreased risk of developing or having bladder cancer or indicative of the bladder cancer stage has changed for the subject;
or
wherein, a determined second RNA aptamer binding profile in the second sample equal to the determined first RNA aptamer binding profile in the first sample is indicative of an unchanged risk of risk of developing or having bladder cancer or that the bladder cancer stage has not changed for the subject;
**characterized in** that the aptamer binding profiles include at least one RNA aptamer comprising a sequence selected from the group consisting of SEQ ID NO: 4 and SEQ ID NO's: 1-3 and 5-34.

In a related to embodiment, a treatment protocol against a bladder cancer has been initiated, continued and/or completed between the sampling of the first and second biological sample.

### Determining effect of bladder cancer treatment

As outlined above, a treatment protocol may be initiated between the time of sampling. Thus, the method of the invention can also be used for determining an effect of a bladder cancer treatment. Therefore, an aspect of the invention relates to a method for determining the effect of a treatment protocol against bladder cancer for a subject, the method comprising
- determining in a first biological sample (1) from the subject, a first RNA aptamer binding profile;
- determining in a second corresponding biological sample from the subject, obtained at a later time point than said first sample, an second RNA aptamer binding profile;
wherein the treatment protocol has been initiated or completed before the sampling of the first sample or initiated, continued or completed between the sampling of the first sample and the second sample; and
wherein, determined overall higher frequencies of the RNA aptamers in the second sample compared the determined frequencies of the RNA aptamers in the first sample, is indicative of the treatment protocol being efficient against the bladder cancer;
or
wherein, determined overall lower or equal frequencies of the RNA aptamers in the second sample compared the determined frequencies of the RNA aptamers in the first sample, is indicative of the treatment protocol being inefficient against the bladder cancer;
**characterized in** that the aptamer binding profiles include at least one RNA aptamer comprising a sequence selected from the group consisting of SEQ ID NO: 4 and SEQ ID NO's: 1-3 and 5-34.

In an embodiment, the method further comprises initiating a treatment or alleviation protocol against invasive or non-invasive bladder cancer, with the proviso that the subject is considered at risk of having or developing invasive or non-invasive bladder cancer.

In a related embodiment, the treatment or alleviation protocol against non-invasive bladder cancer is selected from the group consisting of surgery, such as bladder removal or cystoscopy, chemotherapy, radiation, or combinations thereof.

In yet a related embodiment, the treatment or alleviation protocol against invasive bladder cancer is selected from the group consisting of surgery, such as bladder removal or cystoscopy, chemotherapy, radiation, or combinations thereof.

### Kit of parts

The present invention also relates to a kit of parts. Thus an aspect of the invention relates to a kit of parts comprising
- at least one RNA aptamer selected from the group consisting SEQ ID NO: 1-34
- one or more primers for amplifying the aptamers;
- one or more enzymes for amplifying the aptamers;
- solid supports for sample capture, such as beads; and
- optionally, instructions for using the kit in a method according to the invention.

In an embodiment, the kit comprises at least 2 different RNA aptamers selected from the group consisting of SEQ ID NO: 1-34 such as at least 5 different RNA aptamers, such as at least 10 different RNA aptamers, such as at least 20 different RNA aptamers, such as at least 30 different RNA aptamers, such as at least 34 different RNA aptamers selected from the group consisting of SEQ ID NO: 1-34.

In yet an embodiment, the primer is selected from the group consisting of SEQ ID NO: 35 and 37. Preferably, two primers are included.

### RNA Aptamer

The present invention also relates to the identified aptamers. Thus, an aspect of the invention relates to a composition comprising an RNA aptamer comprising an (aptamer) sequence selected from the group consisting of SEQ ID NO: 4 and SEQ ID NO's: 1-3 and 5-34.

In an embodiment, the RNA aptamer has a length in the range 50-150 nucleotides, such as 60-120 nucleotides, such as 60-100 nucleotides, such as 70-90 nucleotides. The aptamers used in the example section has a length of 80 nucleotides.

The aptamers may comprise one or more artificial nucleotides or bonds. Thus, in an embodiment, the RNA aptamer comprises one more artificial nucleotides and/or artificial bonds.

In yet an embodiment, the artificial nucleotides or artificial bonds are selected from the group consisting of 2'-F-pyrimidine, 2'-NH2-pyrimidine, 2'-O-Me, LNA (locked nucleic acid), 4'-S, TNA (threose nucleic acid), FANA (fluoroarabinonucleotide) and HNA (1,5-anhydrohexitol nucleic acid), preferably 2'-F-pyrimidine. In the example section 2'-F-pyrimidine modified RNA aptamers has been used.

In yet an embodiment, the composition comprises SEQ ID NO: 4. In another embodiment, the composition comprises SEQ ID NO: 4 and/or SEQ ID NO: 34.

### Uses

The kit and compositions according to the invention may have different uses. Thus, an aspect of the invention relates to a kit according to the invention or a composition according to the invention in a method for determining the risk of a subject for having or developing non-invasive bladder cancer or invasive bladder cancer or for staging a bladder cancer.

In an embodiment, the method is an in vitro method, such as performed on a biological sample from a subject, such as a biological sample previously obtained from the subject.

It should be noted that embodiments and features described in the context of one of the aspects of the present invention also apply to the other aspects of the invention.

All patent and non-patent references cited in the present application, are hereby incorporated by reference in their entirety.

The invention will now be described in further details in the following non-limiting examples.

### Examples

### Example 1 - Materials and methods

### SELEX protocol

The 2 '-F-pyrimidine RNA library was prepared as described previously (Dupont et al. RNA. 2010 Dec;16(12):2360-9) with minor modifications. Briefly, dsDNA was prepared by primer annealing and Klenow DNA extension (Thermo Fisher Scientific) using the oligonucleotides 5'-CGCGGATCCTAATACGACTCACTATAGGGGCCACCAACGACATT-3' (forward oligo) (SEQ ID NO: 35 and 5'-GATCCATGGGCACTATTTATATCAAC-N36-AATGTCGTTGGTGGCCC-3' (library oligo) (SEQ ID NO: 36) obtained from Integrated DNA Technologies. dsDNA was purified by 6% non-denaturing PAGE (National Diagnostics) and transcribed in a reaction containing 80 mM HEPES (pH 7.5), 30 mM DTT, 25 mM MgCI2, 2 mM spermidine-HCI, 2.5 mM of ATP and GTP (Thermo Fisher Scientific), 2.5 mM of 2'-F-dCTP and 2'-F-dUTP (TriLink Biotechnologies), 100 µg/mL BSA, 0.5 µM dsDNA template and 150 µg/mL mutant T7 RNA polymerase Y639F. The dsDNA was then digested by DNA polymerase I (Thermo Fisher Scientific) and the 2'-F-pyrimidine RNA library purified by denaturing urea-PAGE.

Thus, an RNA aptamer pool comprising oligonucleotides 80 nt in length is generated with a degenerative sequence of 36 nucleotides.

To select aptamer pools for plasma proteins, either 1 µL of EDTA-plasma or 50 ug of protein material after abundant protein depletion of the plasma (High-Select™ Top14 Abundant Protein Depletion Resin; Thermo Fisher Scientific) was conjugated to 1 mg of NHS-activated magnetic beads (Thermo Fisher Scientific) overnight in HBS (20 mM HEPES pH 7.4, 140 mM NaCI) at 4°C. Plasma derived from patients diagnosed with T2-T4 stage bladder cancer was applied for positive selection. For counter-selection magnetic beads were prepared with BSA only or plasma protein material with or without abundant protein depletion obtained from bladder cancer negative patients (see results section for details). Beads were washed using SELEX wash buffer (SWB; HBS including 2 mM MgCI2, 2 mM CaCI2, 5 mM KCI and 0.01% Tween20) and remaining reactive sites blocked with SWB supplemented with 0.1% BSA for 1 hour. For the first round of selection, the 2'-F RNA library (5 copies of 10^15 different sequences or 8.5 nmol) was refolded by heating to 95 degrees for 2 minutes and cooling on ice and counter-selection performed for 1 hour using SELEX buffer (SB; SWB supplemented with 100 ug/mL tRNA and 0.1% BSA). The supernatant from the counter-selection was subsequently transferred to positive selection magnetic beads and binding allowed for 1 hour (selection). Beads were then washed 3 times with SWB and Superscript III reverse transcription (Thermo Fisher Scientific) performed directly on the washed beads using the reverse oligo 5'-CCCGACACCCGCGGATCCATGGGCACTATTTATATCAA-3' (IDT) (SEQ ID NO: 37). cDNA was converted to dsDNA using the Thermo Scientific Phusion High-Fidelity DNA Polymerase PCR system with forward and reverse oligos, and the optimal number of PCR cycles determined from a small scale reaction prior to the full scale reaction to reduce PCR bias. dsDNA was then digested by BamHI (Thermo Fisher Scientific), purified by the GeneJet PCR purification Kit (Thermo Fisher Scientific), and transcribed to produce RNA for the following round of selection. For subsequent rounds of selection, 200 pmol of RNA pool was applied.
The RNA pools after four rounds of selection were used for branched selection (i.e. one round of parallel selection for each patient sample). The branched selections were performed as described for the aptamer pool selections, however without a counter selection step. For each PCR sample, forward and reverse oligos containing specific barcode sequences were used to allow sample multiplexing during next-generation sequencing. Barcoded PCR samples were submitted to the Beijing Genomics Institute (BGI) for PCR-free library preparation and one-lane 2×100 (Paired End) Illumina HiSeq 4000 sequencing. Reads were subsequently subjected to demultiplexing, pair-mate joining, de-replication and clustering as described previously (Dupont et al. Nucleic Acids Res. 2015 Dec 2;43(21):e139) producing a table of the sequences and the copy numbers observed with each sample. Generally, a total of one to three million read pairs were obtained from each sample. Sequence copy numbers were divided by the total read number to estimate sequence frequency as the fraction of pool in percent allowing comparison of values across samples.

### Example 2 - Pool of aptamers for subtyping bladder cancer

### Aim of study

To determine that the method of the invention can indeed to be used to distinguish healthy individuals (N0), from patients with non-invasive cancer (stage Ta-T1) and distinguish patients with different invasive cancer stages (stage T2-T4) using the 34 identified RNA aptamers.

### Materials and methods

As outlined in example 1, the initial steps of the method described in here is similar to a regular SELEX experiment. Initially, a library containing approximately 10^15 2'-F-stabilized RNAs with random sequences are produced, and the RNA aptamer molecules in the library that bind components in the blood are enriched by 3-4 rounds of SELEX as described in example 1. This process is referred to as "library training". The trained library is subsequently subjected to a single round of selection towards plasma from individual patients in the cohort. This step is referred to as "branched selection". The differential change in the amount of each aptamer is recorded as percentage of the pool and used as input for diagnostic machine learning algorithms and statistical analyses.

The tested aptamers were 2'-fluor-modified (2'-F-pyrimidine modified RNA).

### Results

A trained aptamer pool library for human plasma was produced by four rounds of selection. 32 plasma samples from healthy individuals, 32 samples from patients with non-invasive cancer (stage Ta-T1) and 32 samples from patients with invasive cancer (stage T2-T4), were subjected to branched selection applying only a single microliter of plasma. Using ordinary least squares regression, aptamer levels were investigated and resulted in the identification of 33 aptamers (SEQ ID NO's 1-33, where the amounts differed significantly between patient-groups (see Table 1). The ability of the 33 aptamers to discriminate samples was validated with a new cohort. Figure 1 and figure 2 show the results of the data using a PCA analysis. Figure 3 shows the PLS (partial least squares) characterization of the samples using all 34 aptamers.

In an independent preliminary setup, an additional aptamer (SEQ ID NO: 34) was identified. A higher relative frequency of SEQ ID NO: 34 was shown to be indicative of bladder cancer (data not shown).

### Conclusion

The data demonstrates that the method of the invention has a promising potential for distinguishing healthy and bladder cancer patient. The data also suggest a potential for determining cancer stage.

In sum, the data shows that a subset of individual aptamers have been identified which can be used to distinguish bladder cancer patients from healthy subjects (Figure 1) and distinguish bladder cancer subtypes (Figures 1 and 2). The identified individual aptamer sequences are listed in Table 1 as SEQ ID NO's: 1-34 The best aptamer to distinguish healthy individuals and bladder cancer patients was sequence number 4 (Table 1).

### Sequence listing

**Table 1: 34 RNA aptamers (SEQ ID NO's 1-34) with the ability to distinguish healthy individuals from bladder cancer patients as well as identify stage of bladder cancer. Important conserved regions important for binding in the aptamer sequences are highlighted in bold. Primers and library oligonucleotide are identified as SEQ ID NO's: 35-37.**

| SEQ ID NO | SEQUENCE (5' to 3' direction) |
|---|---|
| 1 | **UUUCC**GCUGAGUAAA**GCGCAG**UCGAGUCUUCC**GACC** |
| 2 | CUUGU**CAUCAG**AAACGCGCUAGUUUC**GCAUUCUG**UC |
| 3 | **UCGGCGUU**GUCAGAAGACCGAAACUUAGACUGUGUU |
| 4 | UUACGGUACGGCUA**AAA**CUG**CACG**ACCU**CUCAGG**CC |
| 5 | UUUAGAAGU**UGAUC**UAAUUCUAA**CUCA**GUACGUCAC |
| 6 | UGAGAUACCAGGCGGUCAU**AUGACA**CCGAAC**UAGU**C |
| 7 | U**UUUCC**CUUCUCAGUAAG**GCAGUCG**UGUUUCAU**GACC** |
| 8 | CGAGCGGUAAAUC**CAA**GCCGC**ACUUC**A**GUC**CGUCAC |
| 9 | UAACGAAUA**UGAUC**CGAUUCGCU**CUCA**GCACAUCAC |
| 10 | CUUAA**CAUCAG**CACAAAUUCUAGUGC**GCAUUCUG**CC |
| 11 | UUGCGACGAUUAUUG**AAA**CCA**CACG**UCGCCUCAGGU |
| 12 | UUUUAGCUC**UGAUC**UAAGCUAAA**CUCA**CGCCAUCCC |
| 13 | GCAUCACCGCUCGACUUACAUCGACCCGUGUAAAAC |
| 14 | UUCGCGCAGAGCUUCAGGUUUCAAUUUAACCCUUUC |
| 15 | AAAUUUCCCUAAAUUGUAGGCAGGUACACCGUACCC |
| 16 | GUCUAACUGUCGGAG**GUACUGUCAACACAUAC**G**AUG** |
| 17 | ACGCGUCUUGGUGACGCUUUUGUGAAUCUAAGCCAC |
| 18 | UUACCUACUCCGUACAUUCGGAUGGUGCCGAACGUU |
| 19 | AGUGUAGGAACUUUCGGACCUCC**AUCAUAAGCAU**GC |
| 20 | GCUUAUCGUGUCUUG**GUACUGUCA**G**CACAUA**CU**AUG** |
| 21 | GGUUUCAAUGUCCCG**GUACUGUCA**U**CACAUAC**A**AUG** |
| 22 | AGCAACUACC**AUGACA**ACGUGU**UAGU**CGGUCGAAAU |
| 23 | AACGUUAAAUGAG**C**C**AAGCGAC**UUGCAUACUUGGAC |
| 24 | CAAAG**CCGAC**AUGCUG**GUGUUCAUGAAUA**CACCAUC |
| 25 | UUA**GGUGCUC**UGAACUUUACAUC**G**G**AACU**AACGUUG |
| 26 | AGUACACCGUAACACUACGGCUU**AUCAUAAGCAU**GAC |
| 27 | UAAAGCGUCGGGCC**GUACUGUCA**AU**ACAUA**CA**AUG** |
| 28 | GUUCGA**UCGGCGUU**AUAAUACCACGCGACCCUUGAC |
| 29 | GC**GGUGCU**UCGCUGCGACAUCCGC**G**A**AAC**CAACGUC |
| 30 | AGUGUGUACGAACAACGUUCGUCCC**AUCAUAAGCAU** |
| 31 | UGAGU**GGUGCUCC**UACAACGCA**G**G**AAC**CUCAAUGCU |
| 32 | **CCGAC**CUUAGUCAAUCACUUC**GUUC**G**AUGAAUA**GCA |
| 33 | UCGUUCGAUCUCUCGAGUUCUCAGAGCGACCUUUAU |
| 34 | AGCACAAAAUUACGCAACCUUGGGACUUAGCUGAUU |
| 35 | CGCGGATCCTAATACGACTCACTATAGGGGCCACCAACGACATT |
| 36 | GATCCATGGGCACTATTTATATCAAC-N36-AATGTCGTTGGTGGCCC |
| 37 | CCCGACACCCGCGGATCCATGGGCACTATTTATATCAA |

Bold indicate conserved regions of the aptamers.

It is to be understood that the aptamer sequences in table 1 (SEQ ID NO's: 1-34), are shown without primer and barcode sequences at the 5'-end and the 3'-end, but are shown with the 36 nucleotide degenerative sequence used for selecting the aptamers and only describe the specific 36 nucleotide random region library sequence for each of the aptamers.

It is also to be understood that preferably the U's and C's are 2 '-fluor-modified (2'-F-pyrimidine modified RNA) in the aptamer sequences in table 1 (SEQ ID NO's: 1-34). Thus, in a preferred embodiment, at least some of the U's and C's are 2 '-F-pyrimidine modified RNA's, preferably all the U's and C's are 2 '-F-pyrimidine modified RNA's. In the example section, the U's and C's in the tested aptamers are 2'-F-pyrimidine modified.

Thus, in a preferred embodiment, the aptamers comprises an additional 5'-sequence and an additional 3'-sequence, such as each having a length in the range 10-50 nucleotides, such as in the range 15-50 nucleotides, preferably 30-50 nucleotides. In a another preferred embodiment, the aptamers comprise an additional 5'-sequence and an additional 3' sequence, such as each having a length in the range 10-60 nucleotides such as in the range 15-50 nucleotides, such as in the range 20-50 nucleotides, preferably 25-40 nucleotides. As shown for the library oligonucleotide (SEQ ID NO: 36) and the primer sequences (SEQ ID NO's: 35 and 37), a certain length may be used at each side of the degenerative sequence. In an embodiment, the forward and/or reverse oligonucleotides contain specific barcode sequences, to allow sample multiplexing during next-generation sequencing.

## Claims

1. A method for determining the risk of a subject for having or developing bladder cancer or for staging a bladder cancer, the method comprising
a) determining for a biological sample from a subject, an RNA aptamer binding profile,
b) comparing the RNA aptamer binding profile to one or more RNA aptamer binding reference profiles;
wherein the comparison to the one or more RNA aptamer binding references profiles, is indicative of the subject being at risk of having or developing invasive bladder cancer or having or developing non-invasive bladder cancer;
or
wherein the comparison to the one or more RNA aptamer binding references profiles, is indicative of a stage of a bladder cancer;
**characterized in that** in step a) the biological sample is mixed with at least one RNA aptamer comprising a sequence selected from the group consisting of SEQ ID NO: 4 and SEQ ID NO's: 1-3 and 5-34.

2. The method according to any of the preceding claims, wherein the biological sample is selected from the group consisting of a blood sample, such as blood serum or blood plasma, a urine sample, a CNF sample, saliva, and a tissue sample, preferably the biological sample is a blood plasma sample.

3. The method according to the any of the preceding claims, wherein the volume of the biological sample is in the range 0.1 µl to 50 µl, such as 0.1 µl to 25 µl, such as 0.1 µl to 10 µl, such as 0.1 µl to 5 µl, such as 0.1 µl to 3 µl, such as 0.5 µl to 2 µl, or such as around 1 µl, preferably a blood plasma sample.

4. The method according to any of the preceding claims, wherein the staging of bladder cancer is staging a non-invasive bladder cancer, such as staging as a Ta or T1 non-invasive bladder cancer or staging an invasive bladder cancer, such as staging as a T2, T3 or T4 invasive bladder cancer.

5. The method according to any of the preceding claims, wherein step a) comprises:
- a1) incubating the biological sample with a surface, thereby enabling coupling of molecules in the biological sample to the surface;
- a2) optionally, washing the surface, thereby removing unbound molecules;
- a3) incubating the surface comprising bound molecules, with at least one RNA aptamer comprising a sequence selected from the group consisting of SEQ ID NO's: 1-34;
- a4) optionally, washing the surface, thereby removing unbound aptamers;
- a5) optionally, releasing the aptamers bound to the surface; and
- a6) determining the identity and optionally frequency of bound aptamers, thereby obtaining an RNA aptamer binding profile.

6. The method according to claim 5, wherein the frequency of the identified aptamers is also determined, such as the relative frequency of the identified aptamers is determined compared to a reference level.

7. The method according to any of the preceding claims, wherein the biological sample is mixed with at least five different RNA aptamers comprising a sequence selected from the group consisting of SEQ ID NO's: 1-34, such as at least 10 different RNA aptamers, such as at least 20 different RNA aptamers, such as at least 30 different RNA aptamers, or such as 34 different RNA aptamers selected from the group consisting of SEQ ID NO's: 1-34.

8. The method according to any of the preceding claims comprising at least SEQ ID NO: 4.

9. The method according to any of the preceding claims wherein
- a lower relative frequency of one or more of SEQ ID NO's: 25, 29 and/or 31 is indicative of invasive bladder cancer; and/or
- a higher relative frequency of one or more of SEQ ID NO's: 16-24, 26-28, 30 and/or 32-33 is indicative of invasive bladder cancer; and/or
- a lower relative frequency of one or more of SEQ ID NO's: 1-15 is indicative of bladder cancer; and/or
- a higher relative frequency of SEQ ID NO: 34 is indicative of bladder cancer.

10. A method for determining an increased, unchanged or decreased risk of developing or having bladder cancer or determining a change in a bladder cancer stage for a subject, the method comprising
• determining in a first biological sample from the subject, a first RNA aptamer binding profile;
• determining in a second corresponding biological sample from the subject, obtained at a later time point than said first sample, an second RNA aptamer binding profile;
wherein, a determined second RNA aptamer binding profile in the second sample different from the first RNA aptamer binding profile in the first sample, is indicative of an increased or decreased risk of developing or having bladder cancer or indicative of the bladder cancer stage has changed for the subject;
or
wherein, a determined second RNA aptamer binding profile in the second sample equal to the determined first RNA aptamer binding profile in the first sample is indicative of an unchanged risk of risk of developing or having bladder cancer or that the bladder cancer stage has not changed for the subject;
**characterized in that** the aptamer binding profiles include at least one RNA aptamer comprising a sequence selected from the group consisting of SEQ ID NO: 4 and SEQ ID NO's: 1-3 and 5-34.

11. A method for determining the effect of a treatment protocol against bladder cancer for a subject, the method comprising
• determining in a first biological sample (1) from the subject, a first RNA aptamer binding profile;
• determining in a second corresponding biological sample from the subject, obtained at a later time point than said first sample, an second RNA aptamer binding profile;
wherein the treatment protocol has been initiated or completed before the sampling of the first sample or initiated, continued or completed between the sampling of the first sample and the second sample; and
wherein, determined overall higher frequencies of the RNA aptamers in the second sample compared the determined frequencies of the RNA aptamers in the first sample, is indicative of the treatment protocol being efficient against the bladder cancer;
or
wherein, determined overall lower or equal frequencies of the RNA aptamers in the second sample compared the determined frequencies of the RNA aptamers in the first sample, is indicative of the treatment protocol being inefficient against the bladder cancer;
**characterized in that** the aptamer binding profiles include at least one RNA aptamer comprising a sequence selected from the group consisting of SEQ ID NO: 4 and SEQ ID NO's: 1-3 and 5-34.

12. A kit of parts comprising
- at least one RNA aptamer selected from the group consisting SEQ ID NO: 4 and SEQ ID NO's: 1-3 and 5-34;
- one or more primers for amplifying the aptamers;
- one or more enzymes for amplifying the aptamers;
- a solid support for sample capture, such as beads; and
- optionally, instructions for using the kit in a method according to any of claims 1-11.

13. A composition comprising an RNA aptamer comprising an (aptamer) sequence selected from the group consisting of SEQ ID NO: 4 and SEQ ID NO's: 1-3 and 5-34.

14. The composition according to claim 13, wherein the RNA aptamer comprises one more artificial nucleotides and/or artificial bonds, such as selected from the group consisting of 2'-F-pyrimidine, 2'-NH2-pyrimidine, 2'-O-Me, LNA (locked nucleic acid), 4'-S, TNA (threose nucleic acid), FANA (fluoroarabinonucleotide) and HNA (1,5-anhydrohexitol nucleic acid), preferably 2'-F-pyrimidine.

15. Use of a kit according to claim 12 or a composition according to claim 13 or 14 in a method for determining the risk of a subject for having or developing non-invasive bladder cancer or invasive bladder cancer or for staging a bladder cancer.
